# EUROPEAN PATENT APPLICATION

(11) **EP 3 381 465 A1**
(43) Date of publication of application: **03.10.2018**
(21) Application number: 18158219.8
(22) Date of filing: 08.07.2015
(51) Int. Cl.: A61K 38/22, A61K 38/08, A61K 9/00, A61P 9/02, A61K 31/137, A61K 47/26

(54) **METHODS FOR TREATING HYPOTENSION**

(30) Priority: 08.07.2014 US 201462022054 P
(62) Divisional of application: 15775357.5
(71) Applicant: La Jolla Pharmaceutical Company, San Diego, CA 92121 (US)
(72) Inventor: TIDMARSH, George, California, 94028 (US); CHAWLA, Lakhmir, California, 92130 (US)
(74) Representative: HGF Limited

(57) **Abstract**

The present disclosure relates to the use of angiotensin II in therapeutic methods for the treatment of hypotension, especially catecholamine-resistant hypotension.

## Description

### Background of the invention

Hypotension, if uncorrected, is life-threatening and occurs as the result of various underlying conditions such as trauma, septic shock or drug reactions. The first line of treatment is intravenous fluids and if this fails to correct the hypotension then vasopressors are deployed. The first line vasopressor is a catecholamine infusion. Catecholamines are amines derived from the amino acid tyrosine, and they include epinephrine (adrenaline), norepinephrine (noradrenaline), phenylephrine, and dopamine, which act as both hormones and neurotransmitters that increase blood pressure. While largely effective at treating hypotension, some patients fail to respond to adequate doses and are defined as catecholamine-resistant. These patients frequently have a high mortality and no acceptable alternatives.

The use of high doses of catecholamines in patients with severe hypotension is associated with poor outcomes. For example, the in-patient, 90-day mortality rate is 50-93% for patients who require norepinephrine as a vasopressor at doses that exceed 0.1 µg/kg/min, and 94% of patients who require norepinephrine at doses above 100 µg/min die.

Thus, alternate methods of regulating blood pressure in patients with catecholamine-resistant hypotension are needed.

### Summary of the invention

Angiotensin II is a peptide hormone naturally produced by the body that regulates blood pressure via vasoconstriction and sodium reabsorption. The hemodynamic effects of angiotensin II administration have been the subject of numerous clinical studies, demonstrating significant effects on systemic and renal blood flow. The invention disclosed herein describes methods of treating hypotension by administering angiotensin II to a patient, measuring the patient's subsequent mean arterial pressure, and titrating the catecholamine and/or angiotensin II dosing being administered to the patient according to the change in the patient's blood pressure.

In some aspects, the invention relates to methods of treating hypotension, such as catecholamine-resistant hypotension, in a patient in need thereof, comprising administering to the patient a composition comprising angiotensin II. The term "catecholamine-resistant hypotension" as used herein refers to patients who require more than 15 µg/kg/min of dopamine, 0.1 µg/kg/min norepinephrine, or 0.1 µg/kg/min epinephrine as a vasopressor. Dopamine, norepinephrine, and epinephrine may be administered at rates higher than 15 µg/kg/min, 0.1 µg/kg/min, or 0.1 µg/kg/min, respectively, but elevated rates correlate with increased mortality.

In some embodiments, the invention relates to methods of treating hypotension in a human patient receiving a catecholamine and having an initial mean arterial pressure, comprising: administering to the patient a composition comprising angiotensin II; after a period of time, measuring the patient's mean arterial pressure; and, if the measured mean arterial pressure is at or above 75 mm Hg, decreasing the rate at which catecholamine is administered to the patient. In certain such embodiments, if the measured mean arterial pressure is below 75 mm Hg, the method comprises increasing the rate of administration of angiotensin II.

The term "mean arterial pressure" or "MAP" refers to the average arterial pressure during a single cardiac cycle.

The term "catecholamine", as used herein, refers to dopamine, norepinephrine, phenylephrine, and epinephrine and their prodrugs, structural analogs, or derivatives that induce similar physiological effects in humans, e.g., raise mean arterial pressure in healthy human subjects. In certain embodiments, the catecholamine may be dopamine, norepinephrine, or epinephrine.

In some embodiments, the invention relates to methods of treating hypotension in a human patient receiving a catecholamine and having an initial mean arterial pressure, comprising: administering to the patient a composition comprising angiotensin II; after a period of time, measuring the patient's mean arterial pressure; and if the measured mean arterial pressure is at or above a threshold value (*e.g.,* 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, or 85 mm Hg), decreasing the rate at which catecholamine is administered to the patient. In certain such embodiments, if the measured mean arterial pressure is below the threshold value, the method comprises increasing the rate of administration of angiotensin II.

In other embodiments, the invention relates to methods of treating hypotension in a human patient receiving a catecholamine and having an initial mean arterial pressure, comprising: administering to the patient a composition comprising angiotensin II; after a period of time, measuring the patient's mean arterial pressure; and if the measured mean arterial pressure is at least 10 mm Hg higher than the initial mean arterial pressure, decreasing the rate at which catecholamine is administered to the patient. In certain such embodiments, if the measured mean arterial pressure is less than 10 mm Hg higher than the initial mean arterial pressure, the method comprises increasing the rate of administration of angiotensin II.

In other embodiments, the invention relates to methods of treating hypotension in a human patient receiving a catecholamine and having an initial mean arterial pressure, comprising: administering to the patient a composition comprising angiotensin II; after a period of time, measuring the patient's mean arterial pressure; and if the measured mean arterial pressure is higher (e.g., at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 mm Hg higher) than the initial mean arterial pressure, decreasing the rate at which catecholamine is administered to the patient. In certain such embodiments, if the measured mean arterial pressure is at or below the initial mean arterial pressure, the method comprises increasing the rate of administration of angiotensin II.

Those of skill in the art will recognize that in the context of the present invention, anti-hypotensive therapeutics can be administered in any suitable way, but are typically administered by continuous infusion. Accordingly, increasing or decreasing a rate of administration can be accomplished by changing the rate of flow of an intravenous drip, changing the concentration of the agent in an intravenous drip, etc. However, the manner in which the rate of administration is changed will depend on the mode of administration of the therapeutic. Where the therapeutic is administered transmucosally or transdermally, the rate may be increased by changing to a higher-release-rate patch or transdermal composition for example. Where the therapeutic is administered orally, the rate may be increased by switching to a higher-dose form, administering additional doses, or administering controlled-release dosage forms with a higher rate of release, for example. Where the therapeutic is administered by inhalation, the rate may be increased by administering additional boluses, a more concentrated bolus, or a faster-release bolus, for example. Other modes of administration (via subcutaneous injection pump, suppository, etc.) can be modulated in analogous fashions, and decreasing the rate of administration can be accomplished by doing the opposite of an action that would increase the rate of administration of the therapeutic.

Angiotensin II may be particularly useful for patients who require potentially harmful doses of vasopressors. Thus, in some embodiments, the invention relates to methods of treating hypotension, wherein, prior to administering the composition, the patient is receiving dopamine, dobutamine, norepinephrine, epinephrine, phenylephrine, terlipressin, vasopressin, or midodrine as a vasopressor.

In some embodiments, the invention relates to methods of treating hypotension wherein the patient has a cardiovascular sequential organ failure assessment score ("SOFA score") of 1 or greater prior to initiation of angiotensin II therapy. For example, a patient may have a cardiovascular SOFA score of 1, 2, 3, or 4. In some embodiments, the patient has a cardiovascular SOFA score of 2, 3, or 4. In other embodiments, the patient has a cardiovascular SOFA score of 3 or 4. In some embodiments, the patient has a cardiovascular SOFA score of 4 prior to initiation of angiotensin II therapy.

In some embodiments, the patient is receiving at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 µg/kg/min of norepinephrine prior to administration of angiotensin II. For example, prior to administering the composition, the patient may be receiving at least 0.1 µg/kg/min of norepinephrine. In other embodiments, wherein the patient may be receiving at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 µg/min of norepinephrine prior to administering the composition.

Alternatively, hypotension may be treated with epinephrine. Thus, in some embodiments, the patient may be receiving at least 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 µg/kg/min of epinephrine prior to initiation of angiotensin II therapy. For example, prior to administering the composition, the patient may be receiving at least 0.1 µg/kg/min of epinephrine. In other embodiments, the patient may be receiving at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 µg/min of epinephrine prior to initiation of angiotensin II therapy..

Alternatively, hypotension may be treated with dopamine. Thus, in some embodiments, the patient may be receiving at least 5, 6, 7, 8, 9, 10, 11, 12, 13, 14, 15, 16, 17, 18, 19, 20, 21, 22, 23, 24, or 25 µg/kg/min of dopamine prior to initiation of angiotensin II therapy. For example, prior to administering the composition, the patient may be receiving at least 5 µg/kg/min of dopamine. In other embodiments, the patient may be receiving at least 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, 1000, 1100, 1200, 1300, 1400, 1500, 1600, 1700, 1800, 1900, 2000, 2100, 2200, 2300, 2400, or 2500 µg/min of dopamine prior to initiation of angiotensin II therapy.

The patient's mean arterial pressure may be monitored to titrate either angiotensin II or the catecholamine. For example, the patient's mean arterial pressure may be monitored with an indwelling arterial line or by other suitable methods. In some embodiments, an initial mean arterial pressure is measured prior to administering the composition, the composition is administered, and, after a period of time, an additional mean arterial pressure is measured. The period of time may be, for example, about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80, 85, 90, 95, 100, 105, 110, 115, 120, 135, 150, 165, 180, 195, 210, 225, or 240 minutes, or about 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24, 25, 26, 27, 28, 29, 30, 31, 32, 33, 34, 35, 36, 37, 38, 39, 40, 41, 42, 43, 44, 45, 46, 47, or 48 hours or longer. Preferably, the period of time is less than two hours, most preferably about one hour or less.

In certain embodiments, if the measured mean arterial pressure meets or exceeds a target value, then the rate at which catecholamine is administered is decreased. The target value may be, for example, 60, 61, 62, 63, 64, 65, 66, 67, 68, 69, 70, 71, 72, 73, 74, 75, 76, 77, 78, 79, or 80 mm Hg. In certain preferred embodiments, if the measured mean arterial pressure is at or above 75 mm Hg, then the rate at which catecholamine is administered is decreased.

In other embodiments, if the difference between the measured mean arterial pressure and the initial mean arterial pressure meets or exceeds a target value, then the rate at which catecholamine is administered is decreased. The target value may be, for example, 1, 2, 3, 4, 5, 6, 7, 8, 9, 10, 11, 12, 13, 14, 15, 16, 17, 18, 19, 20, 21, 22, 23, 24, or 25 mm Hg. In certain preferred embodiments, if the measured mean arterial pressure is at least 10 mm Hg higher than the initial mean arterial pressure, then the rate at which catecholamine is administered is decreased.

The mean arterial pressure may be measured more than once; for example, the mean arterial pressure may be measured 1, 2, 3, 4, 5, 6, 7, 8, 9, 10, or more times, or even continuously or substantially continuously. The rate at which catecholamine is administered may be decreased in response to each measurement (or the rate of angiotensin II decreased, or both), depending on whether the measured mean arterial pressure meets or exceeds a target value. Similarly, the rate at which catecholamine is administered may be increased after a measurement (or the rate of angiotensin II increased, or both), if the measured mean arterial pressure is less than a target value. Similarly, the rate at which catecholamine is administered may be decreased after each measurement (or the rate of angiotensin II decreased, or both), depending on whether the difference between the measured mean arterial pressure and the initial mean arterial pressure is less than a target value. Similarly, the rate at which catecholamine is administered may be increased after a measurement (or the rate of angiotensin II increased, or both), if the difference between the measured mean arterial pressure and the initial mean arterial pressure is less than a target value.

In some embodiments, if the patient's measured mean arterial pressure is at or above 75 mm Hg, then the rate at which catecholamine is administered to the patient is decreased. In some embodiments, if the patient's measured mean arterial pressure is at or above 65, 66, 67, 68, 69, 70, 71, 72, 73, 74, 75, 76, 77, 78, 79, 80, 81, 82, 83, 84, or 85 mm Hg, then the rate at which catecholamine is administered to the patient is decreased. In some embodiments, if the measured mean arterial pressure is at least 10 mm Hg higher than the initial mean arterial pressure, then the rate at which catecholamine is administered to the patient is decreased. In some embodiments, if the measured mean arterial pressure is at least 1, 2, 3, 4, 5, 6, 7, 8, 9, 10, 11, 12, 13, 14, 15, 16, 17, 18, 19, 20, 21, 22, 23, 24, or 25 mm Hg higher than the initial mean arterial pressure, then the rate at which catecholamine is administered to the patient is decreased. In certain embodiments, the rate at which catecholamine is administered is decreased by at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more. Thus, for example, the rate at which norepinephrine is administered is decreased by at least 15%. In other embodiments, the rate at which the catecholamine is administered is decreased by at least 60%. In some embodiments, the rate at which catecholamine is administered is decreased to 0 µg/kg/min.

The catecholamines may be titrated down while monitoring a patient's MAP, and titration may occur over the course of minutes to hours. Thus, the rate at which a catecholamine is administered may be decreased by at least 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9%, or more over the course of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80, 85, 90, 95, 100, 105, 110, 115, 120, 135, 150, 165, 180, 195, 210, 225, or 240 minutes, or over the course of about 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24, 25, 26, 27, 28, 29, 30, 31, 32, 33, 34, 35, 36, 37, 38, 39, 40, 41, 42, 43, 44, 45, 46, 47, or 48 hours or longer.

Angiotensin II is effective at increasing a patient's MAP at concentrations above 1 ng/kg/min. Thus, in certain embodiments, the angiotensin II is administered at a rate greater than or equal to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 ng/kg/min. For example, in some embodiments, the angiotensin II is administered at a rate of 5 ng/kg/min. In other embodiments, the invention relates to methods of treating hypotension wherein angiotensin II is administered at a rate of 20 ng/kg/min. In still other embodiments, the angiotensin II is administered at a rate of 40 ng/kg/min.

Different patients require higher or lower rates of administration of angiotensin II to achieve a treatment goal. The rate of administration may be optimized for different patients by administering angiotensin II at an initial rate and the increasing or decreasing the rate of administration. In some cases, the patient may be administered an initial bolus of angiotensin II followed by the administration of angiotensin II at a lower rate. Alternatively, the patient may be administered angiotensin II at a low rate followed by gradual, elevated rates. Thus, in some embodiments, the method further comprises increasing the rate at which angiotensin is administered, and in other embodiments, the method further comprises decreasing the rate at which angiotensin is administered. For example, angiotensin II may be administered at an initial rate of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10, 11, 12, 13, 14, 15, 16, 17, 18, 19, or 20 ng/kg/min, and the rate may be increased to a final rate of about 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 ng/kg/min. Alternatively, angiotensin may be administered at an initial rate of about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 ng/kg/min, and the rate may be decreased to a final rate of about 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10, 11, 12, 13, 14, 15, 16, 17, 18, 19, or 20 ng/kg/min. Angiotensin II may be titrated while monitoring a patient's MAP, and titration may occur over the course of minutes to hours. Thus, the rate at which at which angiotensin II is administered may be increased or decreased over the course of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 70, 75, 80, 85, 90, 95, 100, 105, 110, 115, 120, 135, 150, 165, 180, 195, 210, 225, or 240 minutes, or over the course of about 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24, 25, 26, 27, 28, 29, 30, 31, 32, 33, 34, 35, 36, 37, 38, 39, 40, 41, 42, 43, 44, 45, 46, 47, or 48 hours or longer.

Angiotensin II may be administered as long as necessary to maintain a MAP above a target value. Alternatively, angiotensin may be administered until the patient's MAP can be maintained at a lower dose of catecholamine. In some embodiments, the composition is administered until the mean arterial pressure of the patient can be maintained at or above 70 mm Hg with less than 0.1 µg/kg/min norepinephrine, less than 0.1 µg/kg/min epinephrine, or less than 15 µg/kg/min dopamine. In other embodiments, the composition is administered continuously over a period of time selected from less than 6 hours; from 6 hours to 24 hours; or at least 24 hours. In other embodiments, the composition is administered continuously for at least 1-6 days, such as 1-11 days.

The methods disclosed herein can use any suitable form or analog of angiotensin II that exhibits the desired effect of increasing MAP in human subjects. In some embodiments, the angiotensin II has the sequence set forth in SEQ ID NO:1, SEQ ID NO:2, SEQ ID NO:3, SEQ ID NO:4, SEQ ID NO:5, SEQ ID NO:6, SEQ ID NO:7, or SEQ ID NO:8. Preferably, the angiotensin II has the sequence set forth in SEQ ID NO:1.

In some embodiments, the angiotensin II is selected from 5-L-valine angiotensin II; 1-L-asparagine-5-L-valine angiotensin II; 1-L-asparagine-5-L-isoleucine angiotensin II; or 1-L-asparagine-5- L-isoleucine angiotensin II, preferably 5-L-isoleucine angiotensin II. The angiotensin II may be formulated as a pharmaceutically acceptable salt, for example, as an acetate salt.

The composition may be formulated with varying concentrations of angiotensin II. Thus, in certain embodiments, the composition comprises angiotensin II at a concentration of about 1, 2, 3, 4, 5, 6, 7, 8, 9, 10, 15, 20, 25, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 550, 600, 650, 700, 750, 800, 850, 900, 950, 1000 µg/ml. In other embodiments, the composition comprises angiotensin II at a concentration of about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, 15.0, 15.5, 16.0, 16.5, 17.0, 17.5, 18.0, 18.5, 19.0, 19.5, 20.0, 20.5, 21.0, 21.5, 22.0, 22.5, 23.0, 23.5, 24.0, 24.5, or 25.0 mg/ml. Thus, in certain embodiments, the composition comprises angiotensin II at a concentration of about 2.5 mg/mL.

In certain embodiments, the composition comprises an excipient, such as mannitol.

In certain embodiments, the composition is suitable for parenteral administration, such as injection or intravenous infusion, preferably intravenous infusion.

In some embodiments, the patient has sepsis. The patient may have septic shock, distributive shock, or cardiogenic shock.

In some embodiments, the patient is a mammal, such as a primate, ovine, porcine, canine, or rodent, preferably a human.

The rate of administration of the angiotensin II can be modulated manually and/or automatically in response to measurements of the patient's mean arterial pressure obtained periodically or sporadically during treatment, *e.g.,* to maintain a mean arterial pressure at this level, or within a predetermined range (*e.g.,* 80-110 mm Hg).

In certain embodiments, the invention provides a method of assessing the response of a patient (such as a human) with hypotension to angiotensin II therapy, comprising administering to the patient an initial dose of a composition comprising angiotensin II (which may be a therapeutic dose or a sub-therapeutic dose, for example, a dose less than 1 ng/kg/min or about 1 ng/kg/min) and testing the patient for a change in a therapeutic parameter (*e.g.,* blood pressure). For example, the therapeutic parameter of the patient can be assessed prior to administering the initial dose and again after administering the initial dose (*e.g.,* at least half an hour later, preferably at least one hour later and/or up to 8 hours later, preferably up to 6 hours later, such as between 1 and 6 hours after administering the initial dose). Comparing the assessment of the therapeutic parameter after administering the initial dose to the assessment made prior to administering the initial dose will indicate whether the parameter is increasing or decreasing as a result of the angiotensin II therapy. Typically, an increase in the patient's blood pressure is indicative of a positive response to the angiotensin II therapy. In certain embodiments, where the patient exhibits a positive response to the therapy, the method further comprises administering an additional dose of angiotensin II to the patient. If a patient exhibits a negative response (*e.g.,* a decrease in the patient's blood pressure), the patient will typically receive no additional doses of angiotensin II therapy. If a patient exhibits no response or an insignificant response, the method may further comprise administering a higher dose of the composition than the initial dose and further testing the patient for a response to the higher dose. Alternatively, if the patient exhibits no response or an insignificant response, the patient may receive no further doses of angiotensin therapy.

### Angiotensin II Therapeutics

Angiotensin II is a peptide hormone naturally produced by the body that regulates blood pressure via vasoconstriction and sodium reabsorption. Hemodynamic effects of angiotensin II administration have been the patient of numerous clinical studies, demonstrating significant effects on systemic and renal blood flow (Harrison-Bernard, L.M., The renal renin-angiotensin system. Adv Physiol Educ, (2009) 33(4): p. 270-74). Angiotensin II is a hormone produced by the renin angiotensin aldosterone system (RAAS) that modulates blood pressure via regulation of vascular smooth muscle tone and extracellular fluid homeostasis. Angiotensin II mediates its effects on the vasculature by inducing vasoconstriction and sodium retention, and so is the target of many therapies for hypertension. In addition to its systemic effects, angiotensin II has a pronounced effect on the efferent arterioles of the kidney, maintaining glomerular filtration when blood flow is decreased. Angiotensin II also regulates sodium reabsorption in the kidney by stimulating Na+/H+ exchangers in the proximal tubule and inducing the release of aldosterone and vasopressin (Harrison-Bernard, L.M., The renal renin-angiotensin system. Adv Physiol Educ, 2009. 33(4): p. 270-4.).

The angiotensin II therapeutic that may be used for in the compositions and methods of this disclosure may be Asp-Arg-Val-Tyr-Ile-His-Pro-Phe(SEQ ID NO: 1) also called 5-isoleucine angiotensin II. SEQ ID NO: 1 is an octa-peptide naturally present in humans and other species, such as equines, hogs, *etc.* Isoleucine may be substituted by valine to result in 5-valine angiotensin II, Asp-Arg-Val-Tyr-Val-His-Pro-Phe(SEQ ID NO: 2). Other angiotensin II analogues such as [Asn¹-Phe⁴]-angiotensin II (SEQ ID NO: 3), hexapeptide Val-Tyr-Ile-His-Pro-Phe (SEQ ID NO: 4), nonapeptide Asn-Arg-Val-Tyr-Tyr-Val-His-Pro-Phe (SEQ ID NO: 5), [Asn¹-Ile⁵-Ile⁸]-angiotensin II (SEQ ID NO: 6), [Asn¹-Ile⁵-Ala⁸]-angiotensin II (SEQ ID NO: 7), and [Asn¹-diiodoTyr⁴-Ile⁵]-angiotensin II (SEQ ID NO: 8) may also be used. Angiotensin II may be synthesized, for example, by solid phase peptide synthesis to incorporate modifications, such as C-terminal amidation. The term "angiotensin II", without further specificity, is intended to refer to any of these various forms, as well as combinations thereof.

The sequence of angiotensin II used in the compositions and methods disclosed herein may be homologous to the sequences of angiotensin II described above. In certain aspects, the invention includes isolated, synthetic, or recombinant amino acid sequences that are at least 80%, 85%, 90%, 95%, 97%, 98%, 99% or 100% identical to SEQ ID NO: 1, 2, 3, 4, 5, 6, 7, and/or 8. Any such variant sequences may be used in place of an angiotensin II as described in the preceding paragraph.

In some aspects, the composition comprising angiotensin II may be selected from 5-valine angiotensin II, 5-valine angiotensin II amide, 5-L-isoleucine angiotensin II, and 5-L-isoleucine angiotensin II amide, or a pharmaceutically acceptable salt thereof, preferably manufactured under current good manufacturing conditions (cGMP). In some aspects, the composition may include different forms of angiotensin II in different percentages, *e.g.,* a mixture of hexapeptide and nonapeptide angiotensin. The composition comprising angiotensin II may be suitable for parenteral administration, *e.g.,* for injection or intravenous infusion.

Similarly, an angiotensin II therapeutic may be used as any suitable salt, deprotected form, acetylated form, deacetylated form, and/or prodrug form of the above-mentioned peptides, including pegylated forms of the peptides or conjugates as disclosed in US Patent Publication 2011/0081371 (incorporated by reference). The term "prodrug" refers to any precursor compound which is able to generate or to release the above-mentioned peptide under physiological conditions. Such prodrugs may be larger peptides which are selectively cleaved in order to form the peptide of the invention. For example, in some aspects, the prodrug may be angiotensinogen, angiotensin I, or its homologues that may result in angiotensin II by the action of certain endogenous or exogenous enzymes. Further prodrugs include peptides with protected amino acids, *e.g.,* having protecting groups at one or more carboxylic acid and/or amino groups. Suitable protecting groups for amino groups are the benzyloxycarbonyl, t-butyloxycarbonyl (BOC), fluorenylmethyloxycarbonyl (FMOC), formyl, and acetyl or acyl group. Suitable protecting groups for the carboxylic acid group are esters such as benzyl esters or t-butyl esters. The present invention also contemplates the use of angiotensin II and/or precursor peptides having amino acid substitutions, deletions, additions, the substitutions and additions including the standard D and L amino acids and modified amino acids, such as, for example, amidated and acetylated amino acids, wherein the therapeutic activity of the base peptide sequence is maintained at a pharmacologically useful level.

### Doses of the therapeutically effective substance

In general, angiotensin II increases blood pressure, and patients who are hypotensive may require larger doses to exhibit pressor responses similar to those observed in normal patients. The composition including the angiotensin therapeutic (*e.g.,* angiotensin II) can be administered at a rate sufficient to achieve an increase in blood pressure of at least about 10-15 mm Hg and optionally for at least angiotensin therapeutic administered may be varied in response to changes in other physiological parameters such as renal vascular resistance, renal blood flow, filtration fractions, mean arterial pressure, *etc.* For example, the rate of administration of the angiotensin therapeutic may start from about 2 ng/kg/min to about 20 ng/kg/min and is increased based on the mean arterial pressure ("MAP"). In some aspects, the rate of administration may be increased such that the MAP does not exceed about 70 mm Hg, about 80 mm Hg, about 90 mm Hg, about 100 mm Hg, about 110 mm Hg, *etc.* For example, a patient may be coupled to a monitor that provides continuous, periodic, or occasional measurements of MAP during some or all of the course of treatment. The rate of administration may be modulated manually (*e.g.,* by a physician or nurse) or automatically (*e.g.,* by a medical device capable of modulating delivery of the composition in response to MAP values received from the monitor) to maintain the patient's MAP within a desired range (*e.g.,* 80-110 mm Hg) or below a desired threshold, *e.g.,* as set forth above.

The composition including the angiotensin therapeutic may be administered over a period of time selected from at least 8 hours; at least 24 hours; and from 8 hours to 24 hours. The composition including the angiotensin therapeutic may be administered continuously for at least 2-6 days, such as 2-11 days, continuously for 2-6 days, for 8 hours a day over a period of at least 2-6 days, such as 2-11 days. A weaning period (from several hours to several days) may be beneficial after prolonged infusion.

The composition including the angiotensin therapeutic may further include one or more additional pharmaceutical agent. For example, angiotensin II may be administered with albumin. The quantity of the additional pharmaceutical agent administered may vary depending on the cumulative therapeutic effect of the treatment including the angiotensin therapeutic and the additional pharmaceutical agent. For example, the quantity of albumin administered may be 1 gram of albumin per kilogram of body weight given intravenously on the first day, followed by 20 to 40 grams daily. Yet other additional pharmaceutical agents may be any one or more of midodrine, octreotide, somatostatin, vasopressin analogue ornipressin, terlipressin, pentoxifylline, acetylcysteine, norepinephrine, misoprostol, *etc.* In some aspects, other natriuretic peptides may also be used in combination with the angiotensin therapeutic to remedy the impairment of sodium excretion associated with diseases discussed above. For example, natriuretic peptides may include any type of atrial natriuretic peptide (ANP), brain natriuretic peptide (BNP), C-type natriuretic peptide (CNP), and/or dendroaspis natriuretic peptide, *etc.* Several diuretic compounds may be used in combination with the angiotensin therapeutic to induce urine output. For example any one or more of the xanthines such as caffeine, theophylline, theobromine; thiazides such as bendroflumethiazide, hydrochlorothiazide; potassium-sparing diuretics such as amiloride, spironolactone, triamterene, potassium canrenoate; osmotic diuretics such as glucose (especially in uncontrolled diabetes), mannitol; loop diuretics such as bumetanide, ethacrynic acid, furosemide, torsemide; carbonic anhydrase inhibitors such as acetazolamide, dorzolamide; Na-H exchanger antagonists such as dopamine; aquaretics such as goldenrod, juniper; arginine vasopressin receptor 2 antagonists such as amphotericin B, lithium citrate; acidifying salts such as CaCl₂, NH₄Cl; ethanol, water, *etc.* may be used in combination with the angiotensin therapeutic to treat the patient. The list of additional pharmaceutical agents described above is merely illustrative and may include any other pharmaceutical agents that may be useful for the treatment of hypotension and related conditions.

### Excipients

The pharmaceutical compositions of the present invention may also contain diluents, fillers, salts, buffers, stabilizers, solubilizers, and other materials well known in the art. The term "pharmaceutically acceptable carrier" refers to a non-toxic carrier that may be administered to a patient, together with a therapeutically effective substance (such as angiotensin II) of this invention, and which does not destroy the pharmacological activity of the therapeutically effective substance. The term "pharmaceutically acceptable" means a non-toxic material that does not interfere with the effectiveness of the biological activity of the active ingredient(s). The characteristics of the carrier will depend on the route of administration. The term "excipient" refers to an additive in a formulation or composition that is not a pharmaceutically active ingredient.

One of skill in the art would appreciate that the choice of any one excipient may influence the choice of any other excipient. For example, the choice of a particular excipient may preclude the use of one or more additional excipients because the combination of excipients would produce undesirable effects. One of skill in the art would be able to empirically determine which excipients, if any, to include in the compositions of the invention. Excipients of the invention may include, but are not limited to, co-solvents, solubilizing agents, buffers, pH adjusting agents, bulking agents, surfactants, encapsulating agents, tonicity-adjusting agents, stabilizing agents, protectants, and viscosity modifiers. In some aspects, it may be beneficial to include a pharmaceutically acceptable carrier in the compositions of the invention.

### Solubilizing agents

In some aspects, it may be beneficial to include a solubilizing agent in the compositions of the invention. Solubilizing agents may be useful for increasing the solubility of any of the components of the formulation or composition, including a therapeutically effective substance (*e.g.,* angiotensin II) or an excipient. The solubilizing agents described herein are not intended to constitute an exhaustive list, but are provided merely as exemplary solubilizing agents that may be used in the compositions of the invention. In certain aspects, solubilizing agents include, but are not limited to, ethyl alcohol, tert-butyl alcohol, polyethylene glycol, glycerol, methylparaben, propylparaben, polyethylene glycol, polyvinyl pyrrolidone, and any pharmaceutically acceptable salts and/or combinations thereof.

### pH-adjusting agents

In some aspects, it may be beneficial to adjust the pH of the compositions by including a pH-adjusting agent in the compositions of the invention. Modifying the pH of a formulation or composition may have beneficial effects on, for example, the stability or solubility of a therapeutically effective substance, or may be useful in making a formulation or composition suitable for parenteral administration. pH-adjusting agents are well known in the art. Accordingly, the pH-adjusting agents described herein are not intended to constitute an exhaustive list, but are provided merely as exemplary pH-adjusting agents that may be used in the compositions of the invention. pH-adjusting agents may include, for example, acids and bases. In some aspects, a pH-adjusting agent includes, but is not limited to, acetic acid, hydrochloric acid, phosphoric acid, sodium hydroxide, sodium carbonate, and combinations thereof.

The pH of the compositions of the invention may be any pH that provides desirable properties for the formulation or composition. Desirable properties may include, for example, therapeutically effective substance (*e.g.,* angiotensin II) stability, increased therapeutically effective substance retention as compared to compositions at other pHs, and improved filtration efficiency. In some aspects, the pH of the compositions of the invention may be from about 3.0 to about 9.0, *e.g.,* from about 5.0 to about 7.0. In particular aspects, the pH of the compositions of the invention may be 5.5±0.1, 5.6±0.1, 5.7±0.1, 5.8±0.1, 5.9±0.1, 6.0±0.1, 6.1±0.1, 6.2±0.1, 6.3±0.1, 6.4±0.1, or 6.5±0.1.

### Buffers

In some aspects, it may be beneficial to buffer the pH by including one or more buffers in the compositions. In certain aspects, a buffer may have a pKa of, for example, about 5.5, about 6.0, or about 6.5. One of skill in the art would appreciate that an appropriate buffer may be chosen for inclusion in compositions of the invention based on its pKa and other properties. Buffers are well known in the art. Accordingly, the buffers described herein are not intended to constitute an exhaustive list, but are provided merely as exemplary buffers that may be used in the compositions of the invention. In certain aspects, a buffer may include one or more of the following: Tris, Tris HCl, potassium phosphate, sodium phosphate, sodium citrate, sodium ascorbate, combinations of sodium and potassium phosphate, Tris/Tris HCl, sodium bicarbonate, arginine phosphate, arginine hydrochloride, histidine hydrochloride, cacodylate, succinate, 2-(N-morpholino)ethanesulfonic acid (MES), maleate, bis-tris, phosphate, carbonate, and any pharmaceutically acceptable salts and/or combinations thereof.

### Surfactants

In some aspects, it may be beneficial to include a surfactant in the compositions of the invention. Surfactants, in general, decrease the surface tension of a liquid composition. This may provide beneficial properties such as improved ease of filtration. Surfactants also may act as emulsifying agents and/or solubilizing agents. Surfactants are well known in the art. Accordingly, the surfactants described herein are not intended to constitute an exhaustive list, but are provided merely as exemplary surfactants that may be used in the compositions of the invention. Surfactants that may be included include, but are not limited to, sorbitan esters such as polysorbates (*e.g.,* polysorbate 20 and polysorbate 80), lipopolysaccharides, polyethylene glycols (*e.g.,* PEG 400 and PEG 3000), poloxamers (*i.e.,* pluronics), ethylene oxides and polyethylene oxides (*e.g.,* Triton X-100), saponins, phospholipids (*e.g.,* lecithin), and combinations thereof.

### Tonicity-adjusting agents

In some aspects, it may be beneficial to include a tonicity-adjusting agent in the compositions of the invention. The tonicity of a liquid composition is an important consideration when administering the composition to a patient, for example, by parenteral administration. Tonicity-adjusting agents, thus, may be used to help make a formulation or composition suitable for administration. Tonicity-adjusting agents are well known in the art. Accordingly, the tonicity-adjusting agents described herein are not intended to constitute an exhaustive list, but are provided merely as exemplary tonicity-adjusting agents that may be used in the compositions of the invention. Tonicity-adjusting agents may be ionic or non-ionic and include, but are not limited to, inorganic salts, amino acids, carbohydrates, sugars, sugar alcohols, and carbohydrates. Exemplary inorganic salts may include sodium chloride, potassium chloride, sodium sulfate, and potassium sulfate. An exemplary amino acid is glycine. Exemplary sugars may include sugar alcohols such as glycerol, propylene glycol, glucose, sucrose, lactose, and mannitol.

### Stabilizing agents

In some aspects, it may be beneficial to include a stabilizing agent in the compositions of the invention. Stabilizing agents help increase the stability of a therapeutically effective substance in compositions of the invention. This may occur by, for example, reducing degradation or preventing aggregation of a therapeutically effective substance. Without wishing to be bound by theory, mechanisms for enhancing stability may include sequestration of the therapeutically effective substance from a solvent or inhibiting free radical oxidation of the anthracycline compound. Stabilizing agents are well known in the art. Accordingly, the stabilizing agents described herein are not intended to constitute an exhaustive list, but are provided merely as exemplary stabilizing agents that may be used in the compositions of the invention. Stabilizing agents may include, but are not limited to, emulsifiers and surfactants.

### Routes of delivery

The compositions of the invention can be administered in a variety of conventional ways. In some aspects, the compositions of the invention are suitable for parenteral administration. These compositions may be administered, for example, intraperitoneally, intravenously, intrarenally, or intrathecally. In some aspects, the compositions of the invention are injected intravenously. One of skill in the art would appreciate that a method of administering a therapeutically effective substance formulation or composition of the invention would depend on factors such as the age, weight, and physical condition of the patient being treated, and the disease or condition being treated. The skilled worker would, thus, be able to select a method of administration optimal for a patient on a case-by-case basis.

Unless otherwise defined herein, scientific and technical terms used in this application shall have the meanings that are commonly understood by those of ordinary skill in the art. Generally, nomenclature and techniques relating to chemistry, molecular biology, cell and cancer biology, immunology, microbiology, pharmacology, and protein and nucleic acid chemistry, described herein, are those well-known and commonly used in the art.

Throughout this specification, the word "comprise" or variations such as "comprises" or "comprising" will be understood to imply the inclusion of a stated integer (or components) or group of integers (or components), but not the exclusion of any other integer (or components) or group of integers (or components). The singular forms "a," "an," and "the" include the plurals unless the context clearly dictates otherwise. The term "including" is used to mean "including but not limited to." "Including" and "including but not limited to" are used interchangeably. The terms "patient" and "individual" are used interchangeably and refer to either a human or a non-human animal. These terms include mammals such as humans, primates, livestock animals (*e.g.,* bovines, porcines), companion animals (*e.g.,* canines, felines) and rodents (*e.g.,* mice, rabbits and rats).

"About" and "approximately" shall generally mean an acceptable degree of error for the quantity measured given the nature or precision of the measurements. Typically, exemplary degrees of error are within 20%, preferably within 10%, and more preferably within 5% of a given value or range of values. Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

### Numbered Clauses

The following numbered clauses provide further embodiments of the invention:
1. A method of treating hypotension in a human patient receiving a catecholamine and having an initial mean arterial pressure, comprising:
   administering to the patient a composition comprising angiotensin II;
   after a period of time, measuring the patient's mean arterial pressure; and
   if the measured mean arterial pressure is at or above 75 mm Hg, decreasing the rate at which catecholamine is administered to the patient.
2. The method of clause 1, further comprising, if the measured mean arterial pressure is below 75 mm Hg, increasing the rate of administering angiotensin II.
3. A method of treating hypotension in a human patient receiving a catecholamine and having an initial mean arterial pressure, comprising:
   administering to the patient a composition comprising angiotensin II;
   after a period of time, measuring the patient's mean arterial pressure; and
   if the measured mean arterial pressure is at least 10 mm Hg higher than the initial mean arterial pressure, decreasing the rate at which catecholamine is administered to the patient.
4. The method of clause 3, further comprising, if the measured mean arterial pressure is less than 5 mm Hg higher than the initial mean arterial pressure, increasing the rate of administering angiotensin II.
5. The method of any preceding clause, wherein the catecholamine is dopamine, norepinephrine, or epinephrine.
6. The method of any one of clauses 1-5, wherein, prior to administering the composition, the patient is receiving at least 0.1 µg/kg/min of norepinephrine.
7. The method of any one of clauses 1-5, wherein, prior to administering the composition, the patient is receiving at least 0.2 µg/kg/min of norepinephrine.
8. The method of any one of clauses 1-5, wherein, prior to administering the composition, the patient is receiving at least 0.5 µg/kg/min of norepinephrine.
9. The method of any one of clauses 1-5, wherein, prior to administering the composition, the patient is receiving at least 1.0 µg/kg/min of norepinephrine.
10. The method of any one of clauses 1-5, wherein, prior to administering the composition, the patient is receiving at least 0.1 µg/kg/min of epinephrine.
11. The method of any one of clauses 1-5, wherein, prior to administering the composition, the patient is receiving at least 0.2 µg/kg/min of epinephrine.
12. The method of any one of clauses 1-5, wherein, prior to administering the composition, the patient is receiving at least 0.5 µg/kg/min of epinephrine.
13. The method of any one of clauses 1-5, wherein, prior to administering the composition, the patient is receiving at least 1.0 µg/kg/min of epinephrine.
14. The method of any one of clauses 1-5, wherein, prior to administering the composition, the patient is receiving at least 5 µg/kg/min of dopamine.
15. The method of any one of clauses 1-5, wherein, prior to administering the composition, the patient is receiving at least 15 µg/kg/min of dopamine.
16. The method of any one of clauses 1-5, wherein, prior to administering the composition, the patient is receiving at least 20 µg/kg/min of dopamine.
17. The method of any one of clauses 1-5, wherein, prior to administering the composition, the patient is receiving at least 25 µg/kg/min of dopamine.
18. The method of any one of clauses 1-5, wherein the patient has a cardiovascular sequential organ failure assessment score ("SOFA score") of 3 or 4.
19. The method of clause 18, wherein the patient has a cardiovascular SOFA score of 4.
20. The method of any one of clauses 1-19, wherein the initial mean arterial pressure is 65 mm Hg or less.
21. The method of any one of clauses 1-19, wherein the initial mean arterial pressure is 70 mm Hg or less.
22. The method of any one of the preceding clauses, wherein the period of time is less than two hours.
23. The method of clause 22, wherein the period of time is about one hour or less.
24. The method of any one of clauses 1-23, wherein the rate at which the catecholamine is administered is decreased by at least 15%.
25. The method of any one of clauses 1-23, wherein the rate at which the catecholamine is administered is decreased by at least 60%.
26. The method of any one of clauses 1-25, wherein angiotensin II is administered at an initial rate greater than or equal to 5 ng/kg/min.
27. The method of any one of clauses 1-25, wherein angiotensin II is administered at an initial rate of about 20 ng/kg/min.
28. The method of any one of clauses 1-25, wherein angiotensin II is administered at an initial rate of about 40 ng/kg/min.
29. The method of any one of clauses 1-28, further comprising the step of increasing the rate at which angiotensin II is administered.
30. The method of clause 29, wherein the rate at which angiotensin II is administered is increased to a final rate of less than or equal to 40 ng/kg/min.
31. The method of clauses 29 or 30, wherein the rate at which angiotensin II is administered is increased over the course of no more than six hours.
32. The method of any one of clauses 1-28, further comprising the step of decreasing the rate at which angiotensin II is administered.
33. The method of clause 32, wherein the rate at which angiotensin II is administered is decreased to a final rate of less than or equal to 5 ng/kg/min.
34. The method of clause 32 or 33, wherein the rate at which angiotensin II is administered is decreased over the course of no more than six hours.
35. The method of any one of clauses 1-34, wherein the composition is administered continuously for at least 1-11 days, such as 1-6 days.
36. The method of any one of clauses 1-34, wherein the composition is administered continuously over a period of time selected from less than 6 hours; from 6 hours to 24 hours; or at least 24 hours.
37. The method of any one of clauses 1-34, wherein the composition is administered until the mean arterial pressure of the patient can be maintained at or above 70 mm Hg with less than 0.1 µg/kg/min norepinephrine, less than 0.1 µg/kg/min epinephrine, or less than 15 µg/kg/min dopamine.
38. The method of any one of the preceding clauses, wherein the angiotensin II has the sequence set forth in SEQ ID NO:1, SEQ ID NO:2, SEQ ID NO:3, SEQ ID NO:4, SEQ ID NO:5, SEQ ID NO:6, SEQ ID NO:7, or SEQ ID NO:8.
39. The method of clause 38, wherein the angiotensin II has the sequence set forth in SEQ ID NO:1.
40. The method of any one of clauses 1-37, wherein the angiotensin II is 5-L-valine angiotensin II; 1-L-asparagine-5-L-valine angiotensin II; 1-L-asparagine-5-L-isoleucine angiotensin II; or 1-L-asparagine-5-L-isoleucine angiotensin II.
41. The method of clause 40, wherein the angiotensin II is 5-L-isoleucine angiotensin II.
42. The method of any one of the preceding clauses, wherein the composition comprises angiotensin II at a concentration of about 2.5 mg/mL.
43. The method of any one of the preceding clauses, wherein the composition further comprises mannitol as an excipient.
44. The method of clause 43, wherein the composition comprises mannitol at a concentration of about 12.5 mg/mL.
45. The method of any one of the preceding clauses, wherein the composition is suitable for parenteral administration.
46. The method of clause 45, wherein the parenteral administration is injection or intravenous infusion.
47. The method of clause 46, wherein the parenteral administration is intravenous infusion.
48. The method of any one of the preceding clauses, wherein the patient has sepsis.
49. The method of clause 48, wherein the patient has septic shock.
50. The method of any one of the preceding clauses, wherein the patient has distributive shock.
51. The method of any one of the preceding clauses, wherein the patient has cardiogenic shock.

### Incorporation by Reference

All publications and patents mentioned herein are hereby incorporated by reference in their entirety as if each individual publication or patent was specifically and individually indicated to be incorporated by reference. In case of conflict, the present specification, including its specific definitions, will control. While specific aspects of the patient matter have been discussed, the above specification is illustrative and not restrictive. Many variations will become apparent to those skilled in the art upon review of this specification and the claims below. The full scope of the invention should be determined by reference to the claims, along with their full scope of equivalents, and the specification, along with such variations.

## Claims

1. A composition comprising angiotensin II, for use in a method of treating hypotension in a human patient receiving a catecholamine and having an initial mean arterial pressure, said method comprising:
administering to the patient a composition comprising angiotensin II;
after a period of time of one hour or less, measuring the patient's mean arterial pressure; and
if the measured mean arterial pressure is at or above 75 mm Hg, decreasing the rate at which catecholamine is administered to the patient;
wherein the method comprises administering angiotensin II at an initial rate of about 20 ng/kg/min.

2. A composition for use according to claim 1, wherein said method further comprises increasing the rate of administering angiotensin II if the measured mean arterial pressure is below 75 mm Hg.

3. A composition comprising angiotensin II, for use in a method of treating hypotension in a human patient receiving a catecholamine and having an initial mean arterial pressure, said method comprising:
administering to the patient a composition comprising angiotensin II;
after a period of time of one hour or less, measuring the patient's mean arterial pressure; and
if the measured mean arterial pressure is at least 10 mm Hg higher than the initial mean arterial pressure, decreasing the rate at which catecholamine is administered to the patient;
wherein the method comprises administering angiotensin II at an initial rate of about 20 ng/kg/min.

4. A composition for use according to claim 3, wherein said method further comprises increasing the rate of administering angiotensin II if the measured mean arterial pressure is less than 5 mm Hg higher than the initial mean arterial pressure.

5. A composition for use according to any preceding claim, wherein the catecholamine is dopamine, norepinephrine, or epinephrine and optionally wherein prior to administering the composition, the patient is receiving at least 0.1 µg/kg/min of norepinephrine, at least 0.1 µg/kg/min of epinephrine, or at least 5 µg/kg/min of dopamine.

6. A composition for use according to any preceding claim, wherein, prior to administering the composition, the patient is receiving at least 0.2 µg/kg/min of norepinephrine.

7. A composition for use according to any preceding claim, wherein the patient has a cardiovascular sequential organ failure assessment score ("SOFA score") of 3 or 4.

8. A composition for use according to claim 7, wherein the patient has a cardiovascular SOFA score of 4.

9. A composition for use according to any preceding claim, wherein the period of time is about 5 minutes.

10. A composition for use according to any preceding claim, wherein the rate at which the catecholamine is administered is decreased by at least 15%.

11. A composition for use according to any preceding claim, further comprising the step of increasing the rate at which angiotensin II is administered, optionally to a final rate of less than or equal to 40 ng/kg/min.

12. A composition for use according to any claims 1 to 10, further comprising the step of increasing the rate at which angiotensin II is administered to a final rate of about 60 ng/kg/min.

13. A composition for use according to any preceding claim, further comprising the step of decreasing the rate at which angiotensin II is administered and optionally wherein the rate at which angiotensin II is administered is decreased to a final rate of less than or equal to 5 ng/kg/min.

14. A composition for use according to any preceding claim, wherein the composition is administered continuously over a period of time selected from less than 6 hours; from 6 hours to 24 hours; or at least 24 hours; and at least 1-11 days, such as 1-6 days.

15. A composition for use according to any preceding claim, wherein the composition is administered until the mean arterial pressure of the patient can be maintained at or above 70 mm Hg with less than 0.1 µg/kg/min norepinephrine, less than 0.1 µg/kg/min epinephrine, or less than 15 µg/kg/min dopamine.

16. A composition for use according to any preceding claim, wherein the composition comprises angiotensin II at a concentration of about 2.5 mg/mL.

17. A composition for use according to any preceding claim, wherein the composition further comprises mannitol as an excipient.

18. A composition for use according to any preceding claim, wherein the pH of the composition is 5.5±0.1.

19. A composition for use according to any preceding claim, wherein the composition is suitable for parenteral administration.

20. The composition for use according to claim 19, wherein the parenteral administration is intravenous infusion.

21. A composition for use according to any preceding claim, wherein the patient has septic shock or distributive shock.
